# FASCICULE DE BREVET EUROPEEN

(11) **EP 2 908 635 B1**
(45) Date de publication et mention de la délivrance du brevet: **05.09.2018**
(21) Numéro de dépôt: 13779591.0
(22) Date de dépôt: 21.10.2013
(51) Int. Cl.: A01N 35/08, A01P 1/00, A61K 35/08

(54) **SUPPORT TEXTILE BIOCIDE**
BIOZIDER TEXTILER TRÄGER
BIOCIDAL TEXTILE SUPPORT

(30) Priorité: 19.10.2012 FR 1259997; 04.12.2012 US 201261733136 P
(43) Date de publication de la demande: 26.08.2015
(73) Titulaire: Paul Boye Technologies, 31810 Vernet (FR)
(72) Inventeur: FOROPON, Valérie, F-09300 Saint Jean d'Aigues Vives (FR)
(74) Mandataire: Lavoix
(86) Numéro de dépôt international: PCT/EP2013/071967
(87) Numéro de publication internationale: WO 2014/060607

(56) Documents cités:
- US-A1- 2006 081 194
- US-A1- 2011 159 304
- US-B1- 6 196 156

## Description

La présente invention est relative à de nouveaux supports biocides, principalement mais non exclusivement textiles, pouvant être utilisés en particulier pour la protection des personnes, comme vêtement ou partie de vêtement, comme accessoire, comme équipement de protection individuel ou pour la protection de locaux.

Cette propriété biocide est conférée selon l'invention par la présence au moins en surface du support, de bronopol ou 2-bromo-2-nitropropane-1,3-diol (CAS 52-51-7) à l'état sec.

Le bronopol est un agent antimicrobien bien connu employé sous forme de solution, qui a trouvé des applications comme conservateur de produits pharmaceutiques ou cosmétiques (EP 1 365 775), comme désinfectant ou agent de conservation dans l'industrie alimentaire (EP 112 100), comme antimicrobien en pisciculture (Pyceze®), comme agent biocide dans des compositions d'embaumement (WO20100010048) ou des compositions pour le traitement des escarres et des blessures externes des cadavres (WO2011033221). Il a aussi été proposé comme agent antimicrobien sur des coussins pour animaux de compagnie (US 6 196 156) ou pour les litières d'animaux de rente (US2006/0081194), ou encore comme agent de protection de pièces métalliques, en mortier ou en béton, enterrées, contre les bactéries réductrices de sulfate.

La présente invention concerne l'intégration, sur et/ou dans un matériau, préférentiellement textile, de bronopol sous forme anhydre. Les résultats des études ayant mené à l'invention ont montré que le bronopol a une activité biocide rapide à l'état sec vis-à-vis des microorganismes, plus précisément, mais non exclusivement, vis-à-vis de nombreuses bactéries et virus pathogènes. Cette activité biocide est apportée par le bronopol à l'état sec à la surface du support ou matériau et en contact physique potentiel avec les microorganismes présents, ce qu'il est convenu d'appeler ici une action biocide contact. Les résultats des études ayant mené à l'invention ont également montré que le bronopol est sans odeur, stable en conditions normales d'utilisation et aussi à des hautes températures pouvant aller jusqu'à 160°C sans que le bronopol ne se dégrade ou se décompose en dérivés dangereux et pouvant être toxiques pour l'homme.

L'invention a donc pour objet un support biocide comprenant du bronopol à l'état sec présent en surface du support et ayant une action biocide contact. Le bronopol est lié au support par un liant photoréticulable ou photopolymérisable. Plus précisément, le bronopol est lié au support par un liant qui a été photoréticulé ou photopolymérisé. Dans le produit fini, soit après réticulation ou polymérisation, le support biocide comprend le bronopol sec lié au support par le produit de la réticulation ou polymérisation d'un liant photoréticulable ou photopolymérisable, et éventuellement par le produit de cette réticulation en présence d'un photoinitiateur. Suivant la composition initiale engagée, d'autres ingrédients peuvent être présents dans le liant réticulé ou polymérisé, par exemple un agent mouillant ou un agent antistatique.

L'invention a aussi pour objet un procédé de préparation d'un tel support, comprenant l'application d'une composition liquide comprenant du bronopol et un liant photoréticulable ou photopolymérisable. Cette composition peut comprendre d'autres ingrédients, notamment un agent mouillant, un agent antistatique, un agent photoinitiateur, comme il sera décrit *infra.* L'invention a aussi pour objet le support susceptible d'être obtenu par la mise en oeuvre de ce procédé et tout article l'incorporant.

Les différentes caractéristiques, particularités, préférences, etc. qui vont être décrites s'appliquent, sauf contre-indication évidente, aux différents objets de l'invention décrits ici.

Dans un mode de réalisation, par support, on entend tout matériau pouvant être imprégné par un substrat, principalement un substrat sous forme aqueuse, mais non exclusivement ; en effet, ce substrat peut aussi être sous forme huileuse ou alcoolisée. Comme matériau répondant à cette définition, on peut citer les mousses, le bois, le cuir, le ciment, à titre d'exemples. On peut également citer les textiles.

Par textile, on entend notamment un tissé, un tricot, un non tissé, une corde ou une tresse.

Dans un mode de réalisation conforme à l'invention, le support est un textile qui est initialement imprégné par une solution de bronopol, puis est séché afin de rendre le bronopol sous un état anhydre.

Le procédé par imprégnation destiné aux supports selon l'invention, notamment textile, comprend les étapes suivantes :
i) trempage du support dans un bain comprenant une composition comprenant du bronopol ;
ii) éventuellement foulardage ou essorage du support issu de l'étape i) ;
iii) séchage du support issu de l'étape i) ou ii).

Dans un mode de réalisation de ce procédé, le support est un textile.

La composition comprenant du bronopol peut être une composition aqueuse. La teneur en poids en bronopol dans la composition, éventuellement aqueuse, peut être supérieure à 0,01%, de préférence comprise entre 0,025 et 20%, avantageusement entre 0,025 et 5%, calculé par rapport au poids total de la composition avant séchage. La composition peut comprendre également un agent mouillant et/ou un agent antistatique.

Comme agent mouillant, on peut citer les alcools gras éthoxylés et les éther-alcools aliphatiques éthoxylés, ou leurs mélanges ; par exemple Invadine® PBN de chez Huntsman comprenant un mélange d'alcool gras éthoxylé et d'éther-alcool aliphatique éthoxylé.

Comme agent antistatique, on peut citer par exemple Ultraphil® TG de chez Huntsman. On peut définir la quantité de bronopol emportée par la quantité de bronopol sec en poids par m² de support. On peut faire varier cette quantité dans de larges proportions, en fonction du niveau de protection biocide recherché, donc en fonction du contexte lié au pouvoir pathogène du microorganisme et de l'environnement dans lequel le personnel sera amené à se déplacer. Cette quantité de bronopol emporté varie en fonction de la capacité du support à absorber la composition, en fonction du réglage de la pression entre les rouleaux exprimeur d'un foulard s'il y a foulardage, en fonction du nombre de passages au foulard réalisés le cas échéant, en fonction de la structure du foulard éventuellement utilisé (nombre de bacs d'imprégnation, circuit du support dans l'équipement...), en fonction des paramètres (temps, vitesse...) de l'essorage réalisé, en fonction du poids du support, de sa composition ou de sa structure. Notamment, le bronopol sera présent sur le support à raison de 0,01 à 50 g, de préférence de 0,05 à 30 g, mieux encore de 0,1 à 20 g par m² de support.

Selon la nature du matériau constituant le support, du bronopol à l'état sec peut être présent à l'intérieur du support et avoir une action biocide contact. Dans une telle circonstance, la quantité de bronopol peut être significativement augmentée et l'on peut exprimer la quantité de bronopol sec par le ratio massique (bronopol/support). Notamment, le ratio massique (bronopol/support) ira de 1/5 000 à 1, de préférence de 1/400 à 1/5.

Dans un second mode de réalisation, le matériau ou support conforme à l'invention, notamment textile, est traité selon le procédé suivant :
a. pulvérisation du support avec une composition comprenant du bronopol ;
b. séchage naturel ou artificiel du support issu de l'étape a)

Par séchage naturel, on entend un séchage à l'air libre du matériau ayant subi la pulvérisation. Par séchage artificiel, on entend tout séchage permettant d'augmenter la vitesse de séchage du matériau, et notamment mais non exclusivement soufflerie d'air chaud, séchage par convecteurs électriques, séchages par rayonnements infrarouge...

La composition comprenant du bronopol peut être une composition aqueuse. La teneur en poids en bronopol dans la composition, éventuellement aqueuse, peut être supérieure à 0,01%, de préférence comprise entre 0,025 et 20%, avantageusement entre 0,025 et 5%, calculé par rapport au poids total de la composition avant séchage. La composition peut comprendre également un agent mouillant et/ou un agent antistatique, comme décrit *supra.*

Dans ce mode de réalisation, la quantité de bronopol pulvérisée peut également être adaptée au niveau de protection biocide recherché, au(x) microorganisme(s) ciblé(s), aux caractéristiques du support utilisé.

Cette quantité est avantageusement comprise entre 0,01 et 50 g par m² de support, de préférence entre 0,05 à 30 g, mieux encore entre 0,1 à 20 g par m² de support.

Dans un troisième mode de réalisation, le matériau ou support, notamment textile, est imprimé, et le bronopol est mélangé à la pâte d'impression (classique), colorée ou non, laquelle est appliquée, puis séchée.

Conformément à l'invention, le bronopol sec (par exemple déposé par imprégnation ou pulvérisation ou impression, notamment comme décrit *supra,* ou par toute autre méthode) est lié au support, notamment textile, par un liant (ou un agent de polymérisation ou de réticulation) ou un mélange de liants. Le support biocide est notamment obtenu par un procédé comprenant les étapes suivantes:
i) soit trempage du support dans un bain comprenant une composition comprenant du bronopol et un liant ou un mélange de liants
ii) soit pulvérisation du support avec une composition comprenant du bronopol et un liant ou un mélange de liants ;
iii) soit impression du support avec une composition comprenant du bronopol et un liant ou un mélange de liants
iv) éventuellement foulardage du support issu de l'étape précédente ;
v) séchage du support issu de l'étape précédente ;
vi) photopolymérisation ou photoréticulation (exposition au rayonnement ad hoc) du liant.

La composition comprenant du bronopol peut être une composition aqueuse. La teneur en poids en bronopol dans la composition, éventuellement aqueuse, peut être supérieure à 0,01%, de préférence elle peut aller de 0,025 à 20%, avantageusement de 0,025 à 5%, calculé par rapport au poids total de la composition avant séchage. La composition peut comprendre également mais non obligatoirement un agent mouillant et/ou un agent antistatique, comme décrit *supra.*

La composition comprend un liant (ou un agent de polymérisation ou de réticulation) par exemple monomère ou polymère, ou un mélange de plusieurs liants. Le liant ou le mélange de liants permet de favoriser la fixation par greffage du bronopol sec à la surface ou dans le matériau du support. Il n'est pas obligatoirement destiné à former une couche continue ou semi-continue à la surface du, ou dans le support. Il peut aussi assurer la fixation ponctuelle du bronopol sec. Le liant ou le mélange de liants permet de former une couche continue ou semi-continue à la surface du, ou dans le, support. Du bronopol sec est accessible en surface de la couche, pour permettre le contact avec les microorganismes à détruire. Le liant est avantageusement choisi parmi des composés chimiques ou des compositions thermoréticulables ou photoréticulables.

On entend notamment par liant thermoréticulable ou thermopolymérisable un liant permettant soit de créer des polymères à partir de monomères ou de monomères et polymères, soit de créer des complexes par ponts ou liaisons chimiques entre polymères, sous l'action de la température.

Un liant photoréticulable ou photopolymérisable sera, en revanche, activé par des rayonnements. On peut citer, à titre d'exemples, les rayonnements ionisants, de type gamma, les rayons X ou les rayons ultra-violets.

On choisira avantageusement les liants photoréticulables ou photopolymérisables parmi les composés vinyliques, les composés acryliques, les amines, les composés polyuréthanes et les matériaux silicone photoréticulables ou photopolymérisables. Le liant est de préférence choisi parmi un composé acrylate, une amine, un composé polyuréthane et un matériau silicone. Il peut notamment s'agir d'un acrylate aliphatique, d'un acrylate hétéroaliphatique, d'un uréthane acrylate, d'un éther acrylate, ou encore d'un mélange d'au moins deux de chaque sorte ou de ces différentes sortes. De préférence, le liant comprend un monomère d'acrylate, l'hexanediol acrylate, le triméthylolpropane triacrylate, le dipropylène glycol diacrylate ou leurs mélanges, photopolymérisables ou photoréticulables.

On peut citer pour les acrylates, notamment les monomère d'acrylate photopolymérisables, par exemple l'Ebecryl LEO® 10501 de chez Allnex, pour l'hexanediol diacrylate, par exemple le Laromer® HDDA de chez BASF, pour le triméthylolpropane triacrylate, par exemple le Laromer® TMPTA de chez BASF et pour le dipropylène glycol diacrylate, par exemple le Laromer® DPGDA de chez BASF. On peut citer, pour les amines, notamment pour les amines photopolymérisables, par exemple l'Ebecryl® P115 de chez Allnex. On peut également citer pour les polyuréthanes, notamment pour une dispersion aqueuse de polyuréthane photopolylmérisable, par exemple l'Ucecoat® 7655 de chez Allnex.

Par conséquent, le support biocide selon l'invention comprendra du bronopol sec et un liant réticulé ou polymérisé issu de la photoréticulation ou photopolymérisation d'un ou plusieurs de ces liants. Le support peut donc comprendre un polymère vinylique, acrylique, aminé ou silicone, notamment un polymère acrylique, et notamment l'un de ceux plus précisément indiqués aux deux paragraphes précédents.

Le polymère acrylique peut notamment être obtenu par polymérisation de monomère acrylate, tel que le dipropylène glycol diacrylate.

Suivant une caractéristique de l'invention, la composition initiale (pour application au substrat) comprenant du bronopol et un liant photoréticulable ou photopolymérisable peut comprendre en outre un liant thermoréticulable ou thermopolymérisable. Comme liant thermoréticulable ou thermopolymérisable, on peut citer par exemple le Printofix binder 83 liq C de chez Clariant. Le support biocide peut donc comprendre du bronopol sec et un liant réticulé ou polymérisé issu d'un double procédé de réticulation ou polymérisation, ou comprenant deux types de liant réticulés ou polymérisés l'un par rayonnement, l'autre par la chaleur.

A noter que dans toute la demande, la notion de liant recouvre aussi la notion de composition polymérisable ou réticulable.

On peut donc aisément comprendre que les quantités de liant vont varier dans de larges proportions en fonction notamment de la quantité de bronopol sec à fixer et à rendre accessible en surface, et des caractéristiques du support traité. Notamment, la teneur en poids en liant dans la composition va de 1 à 80%, de préférence de 2 à 50%, avantageusement de 2 à 20%, calculé par rapport au poids total de la composition avant séchage, puis éventuellement polymérisation ou réticulation.

La composition initiale peut aussi comprendre un photoinitiateur. Le liant photoréticulable ou photopolymérisable peut être activé par rayonnements, notamment ultra-violets (UV), en présence d'un photoinitiateur, par exemple benzophénone. Le liant photoréticulable ou photopolymérisable peut aussi être activé sans photoinitiateur. Notamment, il peut être activé par rayonnements ionisants de type gamma ou les rayons X, en l'absence d'un photoinitiateur.

Le support biocide pourra prendre la forme d'un objet de forme et de surface quelconque. De manière générale, les supports de l'invention sont plutôt des supports plans, flexibles ou rigides, d'épaisseur variable, formant des surfaces, des plaques, des coupons ou des films, par exemple des coupons ou pièces textiles, des pansements ou parties de pansements, des plaques en mousse ou des revêtements de sols ou de murs.

La présente invention a aussi pour objet l'utilisation du bronopol à l'état sec pour conférer des propriétés biocides à un support. Dans cette utilisation, le support présente du bronopol à l'état sec. Il est avantageusement lié au support, notamment comme il vient d'être décrit. Le bronopol sec est en partie au moins au contact de l'air ambiant, pour lui permettre d'exprimer son caractère biocide à l'encontre d'un pathogène susceptible d'entrer en contact avec le support. Le pathogène peut être un microorganisme. Il peut s'agir notamment d'une bactérie, notamment du genre *Bacillus,* de préférence *Bacillus anthracis,* du genre *Staphylococcus,* de préférence *Staphylococcus aureus,* ou du genre *Acinetobacter,* de préférence *Acinetobacter baumanii;* ou d'un virus, par exemple le virus H1N1, l'adénovirus type 3 ou le Parainfluenza 3.

Le support biocide peut être employé en tant qu'élément d'un dispositif ou équipement de protection individuel destiné à la protection contre des risques biologiques.

Il peut notamment être utilisé dans un revêtement ou vêtement ou un équipement de protection individuel ou un accessoire destiné à la protection de l'homme ou l'animal contre un agent pathogène, tel qu'un microorganisme par exemple, préférentiellement un virus ou une bactérie.

Il peut notamment être utilisé dans un revêtement ou vêtement ou un équipement de protection individuel ou un accessoire destiné à la protection de l'homme ou l'animal contre une bactérie, notamment du genre *Bacillus,* de préférence *Bacillus anthracis,* du genre *Staphylococcus,* de préférence *Staphylococcus aureus,* ou du genre *Acinetobacter,* de préférence *Acinetobacter baumanii.*

Il peut notamment être utilisé dans un revêtement ou vêtement ou un équipement de protection individuel ou un accessoire destiné à la protection de l'homme ou l'animal contre un virus, notamment contre un virus influenza, tel que le virus H1N1, un adénovirus, tel que l'adénovirus type 3 ou un virus parainfluenza, tel que le Parainfluenza 3.

L'invention a aussi pour objet un vêtement ou tenue biocide ou un équipement de protection individuel, de préférence antibactérienne ou antivirale, comprenant un support selon l'invention.

L'invention a aussi pour objet un masque, de préférence antibactérien ou antiviral, comprenant un support selon l'invention.

L'invention va être maintenant décrite plus en détail à l'aide de modes de réalisation pris à titre d'exemples. Exemple 1 Un support textile de type tissu est imprégné par foulardage par une solution dont la composition massique est la suivante :

| | |
|---|---|
| Bronopol dilué 1/10 : | 5g |
| Ultraphil® TG : | 0,5g |
| Invadine® PBN | 0,5g |
| Eau : | 94g |

Le support textile est ensuite séché à 160°C pendant 3 minutes.

L'activité bactéricide du textile ainsi traité a été évaluée vis-à-vis de *Bacillus cereus,* selon les indications de la norme JIS Z 2801 :2000

Conditions expérimentales :
- souche de référence : *Bacillus cereus* CIP 105151
- Préparation du support textile : le support est découpé stérilement en portions de 4cmx4cm. Support témoin : boîte de pétri. Les supports sont inoculés avec 400µl de suspension bactérienne de manière à obtenir environ 10⁵ UFC/support, et laissés tels quels durant l'incubation (24h).
- Solutions : les suspensions bactériennes ont été préparées avec la solution Nutrient Broth diluée au 1/250. La solution de récupération est la solution SCDLP préconisée par la norme. Les dilutions ultérieures sont réalisées en PBS (Sigma).
- Milieux : gélose trypcase soja (Biomérieux)
Dépôt sur les supports : 0,84.10⁵ UFC
Le temps de contact est de 24 heures.

**Résultats :**

| | Echantillon testé | UFC |
|---|---|---|
| Témoin à T0 | Boîte de Pétri | 1,03.10⁵ |
| Témoin à T24h | Boîte de Pétri | 1,93.10⁴ |
| Essai à T24h | Tissu traité | < 10 |

Exemple 2 Un support textile en non tissé est imprégné par foulardage avec une solution dont la composition massique est la suivante :

| | |
|---|---|
| Bronopol | 2,5g |
| Invadine® PBN | 0,5g |
| Eau | 197g |

Le non tissé est ensuite séché à 100°C pendant 3 minutes

L'activité bactéricide du textile ainsi traité a été évaluée vis-à-vis de *Staphylococcus aureus,* selon les indications de la norme JIS Z 2801 :2000

Conditions expérimentales :
- souche de référence : *Staphylococcus aureus* ATCC 33591
- Préparation du support textile : le support est découpé stérilement en portions de 4cmx4cm. Support témoin : boîte de pétri. Les supports sont inoculés avec 400µl de suspension bactérienne de manière à obtenir environ 10⁵ UFC/support.
- Solutions : les suspensions ont été préparées avec la solution Nutrient Broth diluée au 1/500. La solution de récupération est la solution SCDLP préconisée par la norme. Les dilutions ultérieures sont réalisées en PBS (Gibco).
- Milieux : gélose trypcase soja (Biomérieux)
- Temps de contact : 24h
Dépôt sur les supports : 1,4.10⁵ UFC
Le temps de contact est de 24 heures.

**Résultats :**

| | Echantillon testé | UFC |
|---|---|---|
| Témoin à TO | Boîte de Pétri | 1,6.10⁵ |
| Témoin à T24h | Boîte de Pétri | 2.10⁴ |
| Essai à T24h | Tissu traité | < 10 |

Exemple 3 Un support textile en non tissé est imprégné par foulardage selon le protocole de l'exemple 2.

L'activité bactéricide du textile ainsi traité a été évaluée vis-à-vis de *Acinetobacter baumanii,* selon les indications de la norme JIS Z 2801 :2000 dans les mêmes conditions expérimentales décrites à l'exemple 2.

La souche de référence : *Acinetobacter baumanii* mR
Dépôt sur les supports : 1,6.10⁵ UFC

Le temps de contact est de 24 heures.

**Résultats :**

| | Echantillon testé | UFC |
|---|---|---|
| Témoin à TO | Boîte de Pétri | 1,5.10⁵ |
| Témoin à T24h | Boîte de Pétri | 1,5.10⁷ |
| Essai à T24h | Tissu traité | < 10 |

On observe que le textile traité selon les exemples 1, 2 et 3 possède une activité bactéricide vis-à-vis des souches bactériennes *Bacillus cereus, Staphylococcus aureus* et *Acinetobacter baumanii.*

Exemple 4 Un support textile en non tissé traité selon l'exemple 2 est également soumis à une évaluation de son activité antivirale vis-à-vis du virus H1N1, de l'Adénovirus type 3 et de Parainfluenza type 3 selon les indications de la norme JIS Z 2801 :2000

### Conditions expérimentales :

- Souche virale de référence : H1N1 ATCC VR-1520
- Souche virale de référence : Adenovirus type 3 ATCC VR-847
- Souche virale de référence : Parainfluenza type3 ATCC VR-93
- Préparation du support textile
   a) le support est découpé stérilement en portions de 4cmx4cm. Support témoin : boîte de pétri en verre.
   b) Préparation de la suspension virale

L'estimation du nombre d'unités infectieuses, c'est-à-dire du titre viral est déterminée par la méthode de SPAERMAN-KÄRBER en calculant le logarithme négatif du point limité 50 % (Ig DICT₅₀).

La technique de titrage est celle indiquée dans la norme NF EN 14476 + A1 (Janvier 2007).

Le titre de la suspension virale est ajusté entre 5,0 Ig DICT₅₀ et 7,5 Ig DICT₅₀ dans du milieu de culture EMEM à 2% SVF (Adénovirus type 3 et Parainfluenza type 3) et EMEM à 0,125 % BSA (H1N1) à partir d'une suspension virale mère selon les recommandations de la norme JIS Z 2801 :2000.

| Suspension virale | Titre suspension virale mère Ig DICT₅₀ | Titre suspension virale ajustée Ig DICT₅₀ |
|---|---|---|
| H1N1 | 9,5 | 7,1 |
| Adénovirus type 3 | 9,3 | 7,3 |
| Parainfluenza type 3 | 9,4 | 7,6 |

### c) Mise en contact virus/supports

- Les supports sont inoculés avec 400 µl de la suspension virale ajustée à l'étape précédente. La suspension virale est recouverte par une lamelle en verre, selon les recommandations de la norme JIS Z 2801 :2000. Conditions d'essai :
- température de contact : 36 ± 1°C
- temps de contact : 24 heures
- Calcul de la réduction logarithme R
   R = Ig DICT₅₀ Essai - Ig DICT₅₀ Témoin 24 heures.

Le traitement du support répond à l'exigence de la norme JIS Z 2801 :2000 si R ≥ 2 Ig.

**Résultats :**

| | Témoin IgDict₅₀ | Essai IgDict₅₀ | R |
|---|---|---|---|
| H1N1 | 4,6 | 0,9 | 3,7 |
| Adénovirus type 3 | 6,3 | 2,5 | 3,8 |
| Parainfluenza type 3 | 5,9 | 3,8 | 2,1 |

Conclusion : dans les conditions de l'essai et selon la norme JIS Z 2801 (version 2000), le textile testé présente une activité virucide (réduction Ig (R) ≥ 2 Ig) vis-à-vis des virus H1N1, Adénovirus type 3 et Parainfluenza type 3 après 24h de contact à 36°C ± 1°C.

Exemple 5 Un textile est imprimé par cylindres rotatifs avec une pâte d'impression dont la composition massique est la suivante :

| | |
|---|---|
| Pâte d'impression pigmentaire : | 1000g |
| Colorant | 28g |
| Bronopol | 20g |
| Dipropylène glycol diacrylate | 100g |

Le textile est ensuite séché et polymérisé 30 secondes à 180°C, puis soumis à des rayonnements gamma.

L'activité bactéricide du textile ainsi traité a été évaluée vis-à-vis de *Bacillus cereus,* selon les indications de la norme JIS Z 2801 :2000

### Conditions expérimentales :

- souche de référence : *Bacillus cereus* CIP 105151
- Préparation du support textile : le support est découpé stérilement en portions de 4cmx4cm. Support témoin : boîte de pétri. Les supports sont inoculés avec 400µl de suspension bactérienne de manière à obtenir environ 10⁵ UFC/support.
- Solutions : les suspensions bactériennes ont été préparées avec la solution Nutrient Broth diluée au 1/250. La solution de récupération est la solution SCDLP préconisée par la norme. Les dilutions ultérieures sont réalisées en PBS (Gibco).
- Milieux : gélose trypcase soja (Biomérieux)
Dépôt sur les supports : 1,11.10⁵ UFC

Le temps de contact est de 24 heures

**Résultats :**

| | Echantillon testé | UFC |
|---|---|---|
| Témoin à TO | Boîte de Pétri | 1,3.10⁵ |
| Témoin à T24h | Boîte de Pétri | 7,3.10⁵ |
| Essai à T24h | Tissu traité | < 10 |

Exemple 6 Un support textile est imprégné par cylindres rotatifs avec une pâte d'impression réactive dont la composition massique est la suivante :

| | |
|---|---|
| Pâte d'impression neutre aqueuse : | 800g |
| Colorant réactif : | 80g |
| Bronopol : | 20g |
| Dipropylène glycol diacrylate: | 100g |
| Benzophénone : | 10g. |

Le textile est séché en rame à 150°C pendant 3 minutes, puis photopolymérisé sous un insolateur UV, et est ensuite introduit dans une vaporiseuse à 102°C pendant 10 min.

Le textile obtenu est rincé à l'eau claire, puis lavé en eau bouillante pendant 3 min et rincé à l'eau tiède.

Le textile lavé est ensuite séché en rame à 150°C pendant 3 minutes.

Le textile obtenu est nommé textile traité et fixé

Un support témoin de type 100% coton est préparé.

La pâte d'impression déposée sur le support textile de référence a une composition massique suivante :

| | |
|---|---|
| Pâte d'impression neutre aqueuse : | 800g |
| Colorant réactif : | 80g |
| Bronopol : | 20g |

Le textile témoin est séché en rame à 150°C pendant 3 minutes et est introduit dans une vaporiseuse à 102°C pendant 10 minutes.

Le textile obtenu est rincé à l'eau claire, puis lavé en eau bouillante pendant 3 min et rincé à l'eau tiède.

Le textile lavé est ensuite séché en rame à 150°C pendant 3 minutes.

Le textile témoin obtenu est nommé textile traité et non-fixé.

L'activité bactéricide du textile traité et fixé, et du textile traité et non-fixé ont été évaluée vis-à-vis de *Bacillus cereus,* selon les indications de la norme JIS Z 2801 :2000 dans les mêmes conditions expérimentales décrites à l'exemple 1.
La souche de référence : *Bacillus cereus* CIP 105151
Dépôt sur les supports : environ 10⁵ UFC
Le temps de contact est de 24 heures

**Résultats :**

| | Echantillon testé | UFC |
|---|---|---|
| Essai à T24h | textile traité et non-fixé | 1,1.10³ |
| Essai à T24h | textile traité et fixé | < 10 |

Conclusion : Le support textile traité et fixé possède une activité bactéricide à T24h contrairement au support textile traité et non-fixé. Le bronopol lié par photopolymérisation au support textile traité et fixé est encore présent après l'étape de lavage sur le support textile traité et fixé tandis que le bronopol non lié par photopolymérisation au support textile traité et non-fixé a été lessivé pendant l'étape de lavage.

Exemple 7 Un support textile de type tissu 100% coton préparé selon le protocole expérimental de l'exemple 6 est soumis à un lavage ménager à 60°C. Le textile ainsi obtenu est nommé un textile traité, fixé et lavé.
Un support textile de type tissu 100% coton préparée selon le protocole expérimental de l'exemple 6 est pris comme support textile témoin. Le textile ainsi obtenu est nommé un textile traité et fixé.

L'activité bactéricide du textile traité, fixé et lavé et du textile traité et fixé ont été évaluée vis-à-vis de *Bacillus cereus,* selon les indications de la norme JIS Z 2801 :2000 dans les mêmes conditions expérimentales décrites à l'exemple 1.
La souche de référence : *Bacillus cereus* CIP 105151
Dépôt sur les supports : 1,11.10⁵ UFC
Le temps de contact est de 24 heures

**Résultats :**

| | Echantillon testé | UFC |
|---|---|---|
| Essai à T24h | textile traité et fixé | < 10 |
| Essai à T24h | textile traité, fixé et lavé | < 10 |

Conclusion : Le bronopol lié par photopolymérisation au support textile traité, fixé et lavé reste présent après un lavage ménager à 60°C. Le support textile traité, fixé et lavé conserve une activité bactéricide à T24h similaire à celle du support textile témoin textile traité et fixé.

Exemple 8 : Rapidité d'action du bronopol à différentes concentrations imprégné sur différents supports textile vis-à-vis de *Bacillus cereus*
Deux supports textile de type polyester/coton sont imprégnés par foulardage respectivement par une solution (1) et une solution (2).
Six supports textile de type polyester/polyamide sont imprégnés par foulardage respectivement par une solution (2), (3), (4) et (5).

Les supports textiles polyester-coton imprégnés par les solutions (1) et (2) sont respectivement nommés textiles polyester/coton traités (1) et (2).

Les supports textiles polyester/polyamide imprégnés par les solutions (2), (3), (4) et (5) sont respectivement nommés textiles polyester/polyamide traités (2), (3), (4), (4A), (5) et (5A).
Les solutions (1), (2), (3), (4) et (5) ont pour composition massique :

| Solution (1): | | Solution (2) : | |
|---|---|---|---|
| Bronopol : | 1g | Bronopol : | 2g |
| Invadine® PBN : | 1g | Invadine® PBN : | 1g |
| Ultraphil® TG : | 1g | Ultraphil® TG : | 1g |
| Eau : | 197g | Eau : | 196g |

| Solution (3) : | | Solution (4) : | |
|---|---|---|---|
| Bronopol : | 3g | Bronopol : | 4g |
| Invadine® PBN : | 1g | Invadine® PBN : | 1g |
| Ultraphil® TG : | 1g | Ultraphil® TG : | 1g |
| Eau : | 195g | Eau : | 196g |

| Solution (5) : | | | |
|---|---|---|---|
| Bronopol : | 10g | | |
| Invadine® PBN : | 1g | | |
| Ultraphil® TG : | 1g | | |
| Eau : | 195g | | |

Les supports textiles polyester-coton sont ensuite séchés à 160°C pendant 2 minutes et les supports textiles polyester/polyamide sont ensuite séchés à 160°C pendant 1 minute.

Le taux d'emport des textiles polyester/coton traité (1) et (2) est de 60%.

Le taux d'emport des textiles polyester/coton est de :
- 126% pour le textile polyester/polyamide traité (2),
- 128% pour le textile polyester/polyamide traité (3),
- 116% pour le textile polyester/polyamide traité (4),
- 127% pour le textile polyester/polyamide traité (4A),
- 116% pour le textile polyester/polyamide traité (5).

L'activité bactéricide des textiles polyester/coton traités (1) et (2) et des textiles polyester/polyamide traités (2), (3), (4), (4A), (5) et (5A) a été évaluée vis-à-vis de *Bacillus cereus,* selon les indications de la norme JIS Z 2801 :2000 dans les mêmes conditions expérimentales décrites à l'exemple 1.
La souche de référence : *Bacillus cereus* CIP 105151
Dépôt sur les supports textiles polyester/coton traités (1) et (2) : 0,64.10⁵ UFC
Dépôt sur les supports textiles polyester/polyamide traités (4) et (5) : 0,41.10⁵ UFC
Dépôt sur les supports textiles polyester/polyamide traités (2), (3), (4A) et (5A) : 0,62.10⁵ UFC

Le temps de contact est de 30 minutes

**Résultats :**

| | Echantillon testé | UFC |
|---|---|---|
| Essai à T0 | textiles polyester/coton traités (1) et (2) | 0,78.10⁵ |
| Essai à T30min | textile polyester/coton traité (1) | ≤10 |
| Essai à T30min | Textile polyester/coton traité (2) | < 10 |
| Essai à T0 | textiles polyester/polyamide traités (4) et (5) | 0,53.10⁵ |
| Essai à T30min | textile polyester/polyamide traité (4) | < 10 |
| Essai à T30min | textile polyester/polyamide traité (5) | < 10 |
| Essai à T0 | textiles polyester/polyamide traités (2), (3), (4A) et (5A) | 0,72.10⁵ |
| Essai à T30min | Textile polyester/polyamide traité (2) | <10 |
| Essai à T30min | Textile polyester/polyamide traité (3) | < 10 |
| Essai à T30min | Textile polyester/polyamide traité (4A) | < 10 |
| Essai à T30min | Textile polyester/polyamide traité (5A) | < 10 |

On observe que les textiles polyester/coton traités (1) et (2) et les textiles polyester/polyamide traités (2), (3), (4), (4A), (5) et (5A) possède une activité bactéricide rapide car en 30 minutes, la souche bactérienne *Bacillus cereus* est détruite.

On observe également que quel que soit le taux d'emport et la quantité de bronopol imprégnée dans ces tissus, ces tissus conservent une activité bactéricide efficace et rapide.

## Revendications

1. Support textile biocide comprenant du bronopol à l'état sec présent en surface du support et ayant une action biocide contact, **caractérisé en ce que** le bronopol est lié, par greffage, au support par un liant photoréticulé ou photopolymérisé.

2. Support biocide selon la revendication 1, **caractérisé en ce que** le bronopol est présent sur le support à raison de 0,01 à 50 g, de préférence de 0,05 à 30 g, mieux encore de 0,1 à 20 g par m² de support

3. Support biocide selon la revendication 1, **caractérisé en ce que** du bronopol à l'état sec est présent à l'intérieur du support et a une action biocide contact.

4. Support biocide selon la revendication précédente, **caractérisé en ce que** le bronopol est présent à l'intérieur du support en un ratio massique (bronopol/support) allant de 1/5 000 à 1, de préférence de 1/400 à 1/5.

5. Support biocide selon l'une quelconque des revendications précédentes, **caractérisé en ce que** le liant comporte un composé vinylique, un composé acrylique, un composé polyuréthane, une amine ou un matériau silicone photoréticulé ou photopolymérisé.

6. Support biocide selon la revendication 5, **caractérisé en ce que** le liant comporte un acrylate aliphatique, un acrylate hétéroaliphatique, un uréthane acrylate, ou un éther acrylate, photoréticulé ou photopolymérisé.

7. Support biocide selon la revendication 5, **caractérisé en ce que** le liant comporte de l'hexandiol diacrylate, du triméthylolpropane triacrylate, du dipropylène glycol diacrylate, photoréticulé ou photopolymérisé..

8. Support biocide selon l'une quelconque des revendications précédentes, **caractérisé en ce que** le bronopol est imprégné ou pulvérisé ou appliqué avec une pâte d'impression sur ou dans le support.

9. Support biocide selon l'une quelconque des revendications précédentes, **caractérisé en ce que** le liant comprend en outre au moins un agent mouillant, un photoinitiateur, ou un agent antistatique.

10. Utilisation d'un support selon l'une quelconque des revendications 1 à 9, en tant qu'élément d'un dispositif ou équipement de protection individuel destiné à la protection contre des risques biologiques.

11. Utilisation selon la revendication 10, dans un revêtement ou vêtement ou un équipement de protection individuel ou un accessoire destiné à la protection de l'homme ou l'animal contre une bactérie, notamment du genre *Bacillus,* de préférence *Bacillus anthracis,* du genre *Staphylococcus,* de préférence *Staphylococcus aureus,* ou du genre *Acinetobacter,* de préférence *Acinetobacter baumanii.*

12. Utilisation selon la revendication 10, dans un revêtement ou vêtement ou un équipement de protection individuel ou un accessoire destiné à la protection de l'homme ou l'animal contre un virus, notamment contre le virus H1N1, l'adénovirus type 3 ou le Parainfluenza 3.

13. Vêtement ou tenue biocide ou un équipement de protection individuel, de préférence antibactérienne ou antivirale, comprenant un support selon l'une quelconque des revendications 1 à 9.

14. Masque, de préférence antibactérien ou antiviral, comprenant un support selon l'une quelconque des revendications 1 à 9.

15. Procédé de fabrication d'un support textile biocide selon l'une quelconque des revendications 1 à 9, comprenant les étapes suivantes:
i) soit trempage du support dans un bain comprenant une composition comprenant du bronopol et un liant ou un mélange de liants
ii) soit pulvérisation du support avec une composition comprenant du bronopol et un liant ou un mélange de liants ;
iii) soit impression du support avec une composition comprenant du bronopol et un liant ou un mélange de liants
iv) éventuellement foulardage du support issu de l'étape précédente ;
v) séchage du support issu de l'étape précédente ;
vi) photopolymérisation ou photoréticulation du liant.

## Patentansprüche

1. Biozider textiler Träger, umfassend Bronopol in trockenem Zustand, das auf der Oberfläche des Trägers vorhanden ist und eine biozide Kontaktwirkung aufweist, **dadurch gekennzeichnet, dass** das Bronopol durch Pfropfen mit dem Träger durch ein fotoretikuliertes oder fotopolymerisiertes Bindemittel verbunden ist.

2. Biozider Träger nach Anspruch 1, **dadurch gekennzeichnet, dass** das Bronopol auf dem Träger im Umfang von 0,01 bis 50 g, vorzugsweise von 0,05 bis 30 g, noch besser von 0,1 bis 20 g pro m² Träger vorhanden ist.

3. Biozider Träger nach Anspruch 1, **dadurch gekennzeichnet, dass** das Bronopol in trockenem Zustand im Inneren des Trägers und mit einer bioziden Kontaktwirkung vorhanden ist.

4. Biozider Träger nach Anspruch 1, **dadurch gekennzeichnet, dass** das Bronopol im Inneren des Trägers in einem Massenverhältnis (Bronopol/Träger) vorhanden ist, das von 1/5000 bis 1, vorzugsweise von 1/400 bis 1/5 reicht.

5. Biozider Träger nach einem beliebigen der vorhergehenden Ansprüche, **dadurch gekennzeichnet, dass** das Bindemittel eine Vinylverbindung, eine Acrylverbindung, eine Polyurethanverbindung, ein Amin oder ein photoretikuliertes oder photopolymerisiertes Silikonmaterial umfasst.

6. Biozider Träger nach Anspruch 5, **dadurch gekennzeichnet, dass** das Bindemittel ein aliphatisches Acrylat, ein heteroaliphatisches Acrylat, ein Urethanacrylat oder ein Etheracrylat, photoretikuliert oder photopolymerisiert, umfasst.

7. Biozider Träger nach Anspruch 5, **dadurch gekennzeichnet, dass** das Bindemittel Hexandioldiacrylat, Trimethylolpropantriacrylat, Dipropylenglycoldiacrylat, photoretikuliert oder photopolymerisiert, umfasst.

8. Biozider Träger nach einem beliebigen der vorhergehenden Ansprüche, **dadurch gekennzeichnet, dass** das Bronopol imprägniert oder pulverisiert oder mit einer Druckpaste auf oder in den Träger aufgetragen ist.

9. Biozider Träger nach einem beliebigen der vorhergehenen Ansprüche, **dadurch gekennzeichnet, dass** das Bindemittel außerdem mindestens ein Benetzungsmittel, einen Photoinitiator oder ein Antistatikum umfasst.

10. Verwendung eines Trägers nach einem beliebigen der Ansprüche 1 bis 9 als Element einer Vorrichtung oder Ausrüstung zum individuellen Schutz, der zum Schutz gegen biologische Risiken ausgelegt ist.

11. Verwendung nach Anspruch 10 in einer Verkleidung oder Kleidung oder einer Ausrüstung zum individuellen Schutz oder einem Zubehör, das für den Schutz eines Menschen oder eines Tiers gegen eine Bakterie, insbesondere der Gattung *Bacillus,* vorzugsweise *Bacillus anthracis,* der Gattung *Staphylococcus,* insbesondere *Staphylococcus aureus,* oder der Gattung *Acinetobacter,* vorzugsweise *Acinetobacter baumanii,* ausgelegt ist.

12. Verwendung nach Anspruch 10 in einer Verkleidung oder Kleidung oder einer Ausrüstung zum individuellen Schutz oder einem Zubehör, das für den Schutz eines Menschen oder eines Tiers gegen einen Virus, insbesondere gegen den Virus H1N1, den Adenovirus Typ 3 oder Parainfluenza 3 ausgelegt ist.

13. Biozide Kleidung oder Aufmachung oder Ausrüstung zum individuellen Schutz, vorzugsweise antibakteriell oder antiviral, umfassend einen Träger nach einem beliebigen der Ansprüche 1 bis 9.

14. Maske, vorzugsweise antibakteriell oder antiviral, umfassend einen Träger nach einem beliebigen der Ansprüche 1 bis 9.

15. Verfahren zur Herstellung eines bioziden textilen Trägers nach einem beliebigen der Ansprüche 1 bis 9, umfassend die folgenden Schritte:
i) entweder Einweichen des Trägers in einem Becken, umfassend eine Zusammensetzung, umfassend Bronopol und ein Bindemittel oder eine Mischung von Bindemitteln,
ii) oder Pulverisieren des Trägers mit einer Zusammensetzung, umfassend Bronopol und ein Bindemittel oder eine Mischung von Bindemitteln;
iii) oder Drucken des Trägers mit einer Zusammensetzung, umfassend Bronopol und ein Bindemittel oder eine Mischung von Bindemitteln
iv) eventuell Klotzen des Trägers, der aus dem vorhergehenden Schritt stammt;
v) Trocknen des Trägers, der aus dem vorhergehenden Schritt stammt;
vi) Photopolymerisieren oder Photoretikulieren des Bindemittels.

## Claims

1. A biocidal textile support comprising bronopol in the dry condition present at the surface of the support and having a biocidal contact action, **characterized in that** the bronopol is bound, by grafting, to the support with a photo-crosslinked or photo-polymerized binder.

2. The biocidal support according to claim 1, **characterized in that** the bronopol is present on the support in an amount from 0.01 to 50 g, preferably from 0.05 to 30 g, still better from 0.1 to 20 g per m² of support

3. The biocidal support according to claim 1, **characterized in that** bronopol in the dry condition is present inside the support and has a biocidal contact action.

4. The biocidal support according to preceding claim, **characterized in that** the bronopol is present inside the support in a mass ratio (bronopol/support) ranging from 1/5,000 to 1, preferably from 1/400 to 1/5.

5. The biocidal support according to any of the preceding claims, **characterized in that** the binder includes a vinyl compound, an acryl compound, a polyurethane compound, an amine or photo-crosslinked or photo-polymerized silicon material.

6. The biocidal support according to claim 5, **characterized in that** the binder includes an aliphatic acrylate, heteroaliphatic acrylate, a urethane acrylate, or an ether acrylate, either photo-crosslinked or photo-polymerized.

7. The biocidal support according to claim 5, **characterized in that** the binder includes hexanediol diacrylate, trimethylolpropane triacrylate, dipropylene glycol diacrylate, either photo-crosslinkable or photo-polymerizable.

8. The biocidal support according to any of the preceding claims, **characterized in that** the bronopol is impregnated or sprayed or applied with a printing paste on or in the support.

9. The biocidal support according to any of the preceding claims, **characterized in that** the binder further comprises at least one wetting agent, one photo-initiator or one antistatic agent.

10. The use of a support according to any of the preceding claims, as an element of a device or piece of equipment for individual protection intended for protection against biological hazards.

11. The use of according to claim 10, in a coating or garment or a piece of individual protection or an accessory intended for protecting humans or animals against a bacterium, notably of the genus *Bacillus,* preferably *Bacillus anthracis,* of the genus *Staphylococcus,* preferably *Staphylococcus aureus,* or of the genus *Acinetobacter,* preferably *Acinetobacter baumanii.*

12. The use according to claim 10, in a coating or garment or a piece of individual protection or an accessory intended for protecting humans or animals against a virus, natively against the H1N1 virus, the adenovirus of type 3 or the Parainfluenza 3 virus.

13. Biocidal clothing or garment or a piece of individual protection, preferably antibacterial or antiviral, comprising a support according to any of claims 1 to 9.

14. A mask, preferably antibacterial or antiviral, comprising a support according to any of claims 1 to 9.

15. A method for manufacturing a biocidal textile support according to any of claims 1 to 9, comprising the following steps:
i) either soaking the support in a bath comprising a composition comprising bronopol and a binder or mixture of binders;
ii) or spraying the support with a composition comprising bronopol and a binder or mixture of binders;
iii) or printing the support with a composition comprising bronopol and a binder or a mixture of binders;
iv) optionally padding the support from the preceding step;
v) drying the support from the preceding step;
vi) photopolymerizing or photo-crosslinking of the support-dried composition complex.
